# EUROPEAN PATENT SPECIFICATION

(11) **EP 4 507 569 B1**
(45) Date of publication and mention of the grant of the patent: **01.07.2026**
(21) Application number: 23720261.9
(22) Date of filing: 14.04.2023
(51) Int. Cl.: A61B 5/021, A61B 5/022

(54) **WEARABLE BLOOD PRESSURE MONITORING DEVICE**
WEARABLE-BLUTDRUCKMESSÜBERWACHUNGSVORRICHTUNG
DISPOSITIF PORTATIF DE SURVEILLANCE DE LA PRESSION ARTÉRIELLE

(30) Priority: 15.04.2022 EP 22305561
(43) Date of publication of application: 19.02.2025
(73) Proprietor: Hinlab, 92200 Neuilly-Sur-Seine (FR)
(72) Inventor: DE BEGON DE LAROUZIERE DE MONTLOSIER, Denys- Michel, 92200 NEUILLY-SUR-SEINE (FR)
(74) Representative: Icosa
(86) International application number: PCT/EP2023/059780
(87) International publication number: WO 2023/198883

(56) References cited:
- US-A1- 2010 331 709
- US-A1- 2011 066 010
- US-A1- 2020 155 409
- US-A1- 2021 393 151

## Description

### FIELD OF INVENTION

The present invention relates to the general field of blood pressure monitoring devices.

### BACKGROUND OF INVENTION

Wearable monitoring devices have gained growing interest as they allow to collect and deliver in real-time various physiological parameters of the wearer.

Wearable blood pressure monitoring devices known in the art generally comprise an inflatable bag connected to a pressure sensor and a pump; the bag being disposed around a limb of the subject and configured to be confined in a predefined volume during a measurement. An example of such wearable blood pressure monitoring device is disclosed in document US 2021/0393151. The blood pressure (systolic and/or diastolic) of the subject may be computed using the known oscillometric method from the continuously recorded pressure measurements during inflation and/or deflation of the bag.

Such devices generally comprise a casing where the pump and electronic components of the device are located, and a soft band where the inflatable bag is placed. The casing may be directly fixed to the soft band on an outside wall thereof, or be external to the soft band.

When the device is fixed to the outside wall of the soft band, a complex connection has to be provided between the hard casing and the bag in the soft band, resulting in a device which is bulky.

When the device is external to the soft band, the pump in the casing is connected to the bag via a flexible tubing, which results also in a bulky device which is not suitable for being worn.

Thus, there is a need for a wearable blood pressure monitoring device with minimal space requirement and that is very convenient to use.

### SUMMARY

This invention thus relates to a wearable blood pressure monitoring device comprising:
- a cuff configured to be worn around a limb of a user, said cuff extending along a longitudinal direction;
- an inflatable compartment located in the cuff and configured to be inflated or deflated; and
- a casing connected to the cuff and comprising a pressure management unit configured to inflate or deflate the inflatable compartment and measure a pressure therein,
wherein the inflatable compartment has a wall transversal to the longitudinal direction and the casing has a lateral wall transversal to the longitudinal direction comprising at least one hole, the wearable blood pressure monitoring device further comprising at least one pneumatic connector extending parallel to the longitudinal direction between the wall of the inflatable compartment and the hole of the lateral wall of the casing to connect the inflatable compartment to the pressure management unit.

The pneumatic connector may comprise a tubing or pipe.

Thanks to the connection of the inflatable compartment, e.g. a bladder, between a wall of the bladder extending in a transversal direction of the cuff and a lateral wall of the casing, there is a minimal space requirement in a vertical direction perpendicular to the longitudinal and transversal direction. This configuration also allows the cuff to be properly wrap around the limb of the subject leading to a better fixation of the device on the limb of the subject, and more repeatable and accurate pressure measurements.

Whereas, in the prior art wearable devices, the space requirement is higher because the pneumatic connection between the bag and the pump extends either in the transversal direction or in the vertical direction.

Moreover, such positioning of the pneumatic connection between the inflatable compartment and the casing allows to include the casing inside the cuff, at least partially. For example, the lateral walls of the casing may be located inside the cuff (i.e., between two opposites layers of the cuff). Said differently, the casing can be at least partially integrated in or positioned within or encapsulated in the cuff. In other embodiments, the casing may be connected to the cuff (or the part of the cuff enclosing the bladder when the cuff is in two separate parts) near the lateral wall of the casing such that only the lateral wall of the casing is located inside the cuff (or the part of the cuff enclosing the bladder when the cuff is in two separate parts).

Such arrangement also allows to have the casing near the skin of the user. For example, the casing may have a bottom wall that is at least partially in contact with the skin of the user when the device is worn. It is particularly convenient when other sensors configured to measure a physiological parameter of the user are used (e.g. an optical sensor, as described below) and located directly in the casing proximate the skin of the user. Indeed, the use of connection sheets in the cuff, which would complexify the conception of the device and reduce its robustness, is not necessary.

In particular, the casing may house a processing unit and at least one sensor configured to measure a physiological parameter of the user. The cuff may comprise a bottom side configured to face the limb of the user and the casing may have a bottom wall extending transversally to the lateral wall. The casing may be attached to the cuff such that at least a part of the bottom wall of the casing is flush with the bottom side of the cuff so as to be in contact with the limb of the user when the wearable blood pressure monitoring device is worn.

According to an embodiment, the device may be worn at an upper limb (e.g. around a forearm, an arm or a wrist) or a lower limb (e.g. around an ankle). When provided at a wrist, the device may be in the form of a watch.

The casing may comprise first and second lateral walls facing each other and connected to each other by the bottom wall, and the cuff may extend in the longitudinal direction from an attachment edge connected to the first lateral wall of the casing, the second lateral wall of the casing being configured to secure a part of the cuff distant from the attachment edge in the longitudinal direction.

The bottom wall may have an aperture and said at least one sensor may comprise an optical sensor arranged in the casing in correspondence with the aperture so as to acquire a signal representative of blood pulse features at the limb of the user.

The bottom wall may comprise a protrusion configured to accommodate the optical sensor, the aperture being arranged in the protrusion. The protrusion provides better contact between the optical sensor and the limb of the user. The protrusion reduces the space, in use, between the limb of the subject and the optical sensor to improve the quality of the signal measured.

The processing unit may be configured to calculate a blood pressure of the user. The blood pressure may comprise a systolic blood pressure and a diastolic blood pressure.

Said at least one sensor may comprise at least one of: an accelerometer (Acc), a gyroscope (Gyr), at least one electrode (Elec), an ultrasound sensor, a tomographic sensor, an acoustic sensor (Mic), a magnetometer, a humidity sensor, a temperature sensor (T), a heat-flux sensor (Htf).

According to an embodiment, the pneumatic connector may have a retainer cooperating with the hole so as to secure the inflatable compartment to the casing. Such retainer provides a stable pneumatic connection between the inflatable compartment and the pressure management unit (e.g. a pump or pressure sensor) and ensure that the inflatable compartment will not move during use of the device, thus improving the quality of the measurements and the lifetime of the device.

The pneumatic connector may have a general cylindrical shape and the retainer may comprise at least a maintaining wing positioned around the pneumatic connector to secure the connector to the casing.

According to an embodiment, the wall of the inflatable compartment may face the lateral wall of the casing.

According to an embodiment, the inflatable compartment may comprise two pneumatic connectors spaced apart in a transversal direction and the casing may comprise two corresponding holes. Using two connectors transversally separated allows to ensure a better securing of the bladder to the casing, thus avoiding unwanted movements of the inflatable compartment in the cuff, for example rotation of the inflatable compartment in the cuff is avoided.

According to an embodiment, the pressure management unit may comprise a pressure sensor connected to one of the two pneumatic connectors, a pump connected to the other of the two pneumatic connectors, and a control unit for acquiring signals from the pressure sensor and for controlling the pump. With this configuration, the different functions of the pressure management units are decoupled, and allows to get better pressure measurements in particular when the pump is activated (e.g., during oscillometric method blood pressure measurement). The pump may itself comprise a valve to control inflation/deflation the bladder or a separate controlled valve may be provided. For example, said separate controlled valve may be connected via a Y connector to the pump and one of the pneumatic connectors of the inflatable compartment.

According to an embodiment, the inflatable compartment may be made from a single sheet of material bent back on itself and bonded along its edges, the at least one pneumatic connector being located near or on a folding line of said sheet. This particular bladder shows good resistance to deformations and is easy to make.

According to an embodiment, the cuff may comprise a bottom side configured to face the limb of the user and an upper side opposite to the bottom side, and wherein the casing is located in the cuff and comprises a bottom wall leading to the bottom side of the cuff, and an upper wall leading to the upper side of the cuff. This configuration improves the compactness of the device by hiding the casing inside the cuff. The cuff may comprise a first layer as its bottom side and a second layer as its upper side. The cuff may comprise a pocket in which the casing is positioned.

According to an embodiment, the casing comprises at least one peripheral flange at the periphery of the bottom wall or the upper wall, said flange being bonded to the cuff. This configuration allows a smooth transition between the cuff and the casing and avoids introduction of contaminants like liquids or particles inside the cuff.

According to an embodiment, the casing and the cuff may be water leak tight, and the upper wall of the casing comprises at least one aperture and a porous waterproof membrane closing said aperture in order to permit gas to enter or exit the casing.

According to an embodiment, the casing may comprise at least a bottom part comprising the bottom wall and an upper part comprising the upper wall, wherein the bottom part and the upper part are assembled with snap-fit fixations located inside the casing. This type of fixation permits an easy mounting of the device.

According to an embodiment, the casing may comprise at least a bottom part comprising the bottom wall and an upper part comprising the upper wall, and may further comprise a gasket between the bottom part and the upper part, said gasket being bonded to the cuff. The gasket provides a better seal of the device and a smoother, more flexible and stronger linkage between the casing and the cuff.

Preferably, the optical sensor is leading to the bottom wall of the casing, when present (to face the limb of the subject). The optical sensor may be a photoplethysmography (PPG) sensor. The optical sensor may comprise a green LED, a red LED, an infrared LED, and at least one photodiode. The signals measured by the optical sensor may then be processed by a processing unit to obtain measurements like heart rate, pulse rate, heart rate variability, respiration rate, blood pressure, blood pressure trend, pulse wave velocity, oxygen saturation (SaO2 or SpO2), and the like.

According to an embodiment, the cuff may comprise two layers of material bonded together along their edges so as to form an internal volume housing the inflatable compartment and the casing. For example, the material may be a polyamide fabric.

Another object of the invention relates to a method of monitoring blood pressure of a user comprising: providing a device as described previously, attaching said device to a limb of the user, and obtaining a blood pressure of the user using said device.

### DEFINITIONS

In the present invention, the following terms have the following meanings:

The terms *"adapted"* and *"configured"* are used in the present disclosure as broadly encompassing initial configuration, later adaptation or complementation of the present device, or any combination thereof alike, whether effected through material or software means (including firmware).

The terms *"processor"* (or *"processing unit*") should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### BRIEF DESCRIPTION OF THE DRAWINGS

The invention will be better understood with the attached figures, in which:
**Figure 1A** is a perspective view of a device according to an embodiment of the invention, wherein the device is wrapped.
**Figure 1B** is another perspective view of the device of figure 1A.
**Figure 2** is a perspective view of the figure of figures 1A and 2B, wherein the device is positioned flat.
**Figure 3** shows two views of the layers constituting the cuff in a preferred embodiment of the invention.
**Figure 4** shows how an inflatable compartment and a casing are assembled in an embodiment of the invention.
**Figure 5** shows two pneumatic connectors used in the inflatable compartment, according to an embodiment of the invention.
**Figure 6** shows a cross-sectional view according to the plane VI-VI of figure 2.
**Figure 7** shows an exploded perspective view of the casing according to an embodiment of the invention.
**Figure 8** shows a view similar to figure 6 of an alternate embodiment of the device.
**Figures 9** and **Figure 10** show respectively a perspective view of a casing and a cross-sectional view similar to Figures 6 and 8, of another alternate embodiment of the device.
**Figure 11** shows a device according to another embodiment, where the cuff is connected to the casing only at one side thereof.
**Figure 12** shows a device according to another embodiment, where the cuff is in two parts respectively connected to two opposite sides of the casing.
**Figure 13** shows a device according to another embodiment, where the casing is positioned in a pocket inside the cuff.
**Figure 14** shows a perspective view of the components inside the casing.
**Figure 15** is an enlarged view of a protrusion on the casing and an optical sensor therein.
**Figure 16** is schematic representation of a device according to an embodiment of the invention.

### DETAILED DESCRIPTION

The present description illustrates the principles of the present disclosure.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, *i.e.,* any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

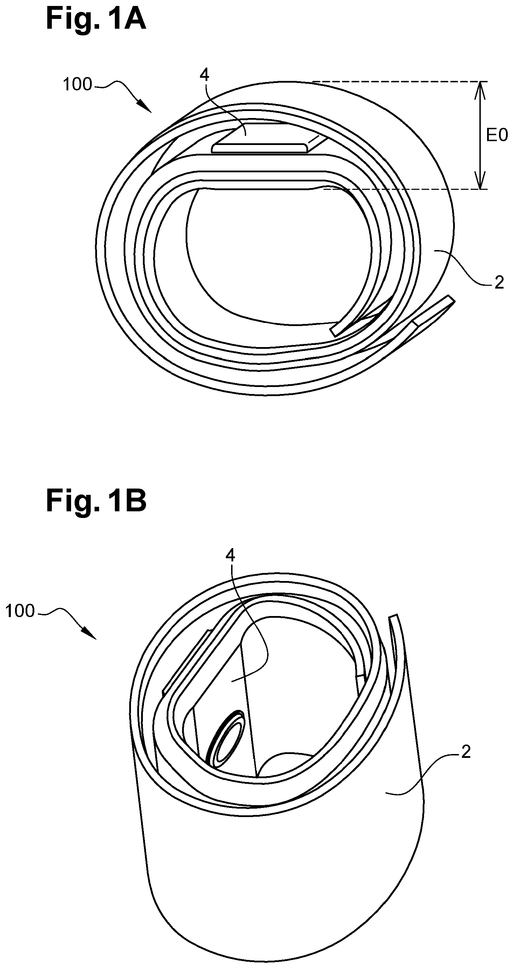

**Figure 1A and Figure 1B** show perspective views of a wearable blood pressure monitor 1 according to an embodiment of the invention, in a configuration where it is wrapped. In use, the device 100 is wrapped around a limb of the subject the way shown in this figure. The limb may be preferably an upper arm.

The device compactness may be appreciated with the thickness E0 of the device 100 when it is wrapped around a limb of a subject (e.g. an upper arm), it may range from 10 mm to 40 mm. This thickness may of course vary when the device is designed for another limb of the subject, for example a wrist.

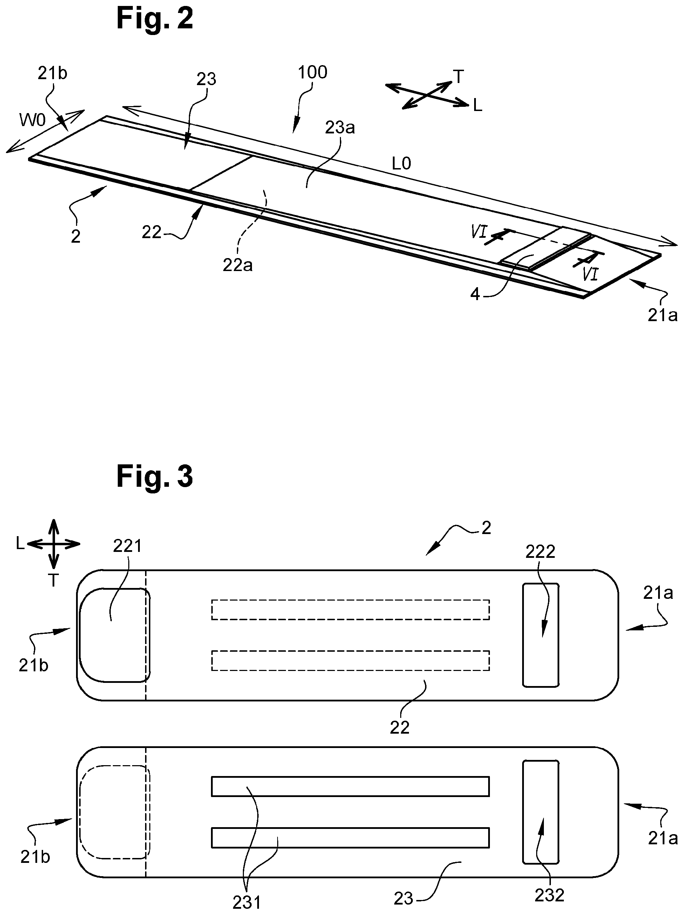

The device 100 generally comprises a cuff 2, a bladder 3 (or inflatable compartment, see Figure 3) and a casing 4. In this example, the casing 4 is partially embedded in the cuff 2, i.e. the casing 4 is present inside the cuff 2 and has at least one wall totally or partially exposed to the outside of the cuff 2. In other embodiments, the casing 4 may be totally embedded inside the cuff 2, i.e. the casing 4 is hidden inside the cuff 2.

The casing 4 houses electronic parts and different sensors of the device 100, which sensors are configured to measure a physiological parameter of the user. The cuff 4 is flexible and encloses the bladder 3 and at least part of the casing 4 to protect them from the outside. The compactness of the assembly between the casing 4 and the cuff 4 allows to wrap easily the device 100 around the arm of a user.

The cuff generally extends in a longitudinal direction L (i.e. the direction of greater dimension) and in a transversal direction T perpendicular to the direction L (i.e. the width of the cuff). As can be seen in Figure 2, the casing 4 may be located in the vicinity of a first extremity 21a of the cuff 2. The cuff 2 comprises a bottom layer 22 comprising a bottom side 22a and an upper layer 23 comprising an upper side 23a. The bottom side 22a is, when the device 100 is in use, facing the limb of the subject. The upper side 23a is opposite the bottom side 22a.

The cuff 2 may be made using two sheets of material forming the two layers 22 and 23. The material may be a fabric, for example a polyamide fabric. The two layers 22 and 23 are shown separated in **Figure 3****.** The layers 22 and 23 may be joined together at their edges by any method known in the art, like sewing, stitching or welding.

The bottom layer 22 extends longitudinally between the first extremity 21a and a second extremity 21b of the cuff 2. The bottom layer 22 comprises a scratch portion 221 (for example of Velcro^{®} band type) near the second extremity 21b, and an aperture 222 near the first extremity 21a. The upper layer 23 also extends between the first extremity 21a and the second extremity 21b. The upper layer 23 comprises two parallel scratch bands 231 (alternatively one or more than two bands may be used) extending longitudinally and configured to be removably attached to the scratch portion 221 of the bottom layer 22 when the device 100 is wrapped around the limb of a user.

The upper layer 23 here comprises an aperture 232 positioned substantially in line with the aperture 222 of the bottom layer 22. Thus, the apertures 222 and 232 are configured to be superposed when the cuff 2 is assembled, and are sized to let appear at least partially the bottom 35 and upper 36 walls of the casing

In alternative embodiments, the cuff 2 may be made from only one sheet of material that is bend over itself and joined at its free edges.

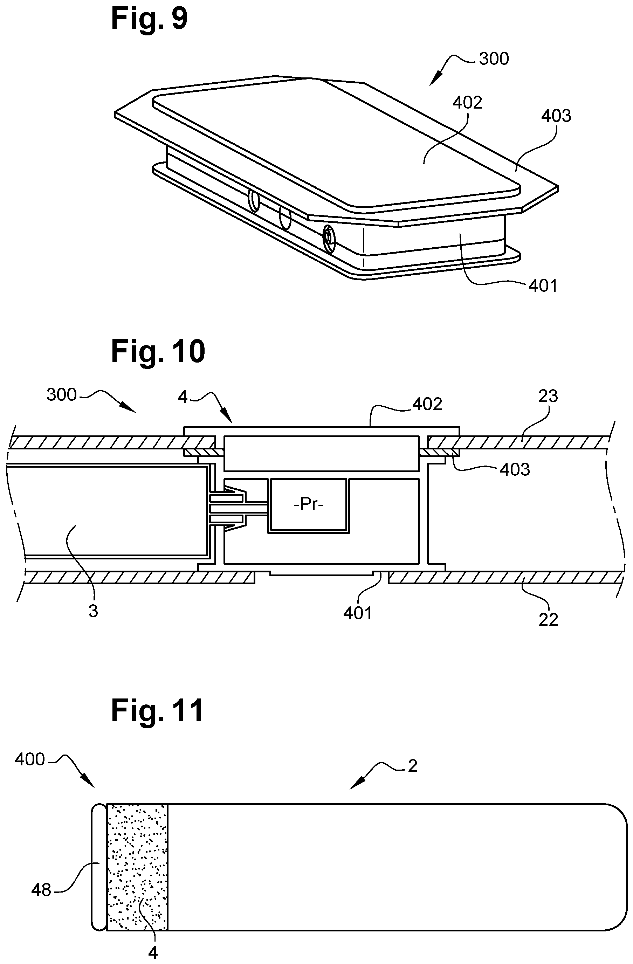

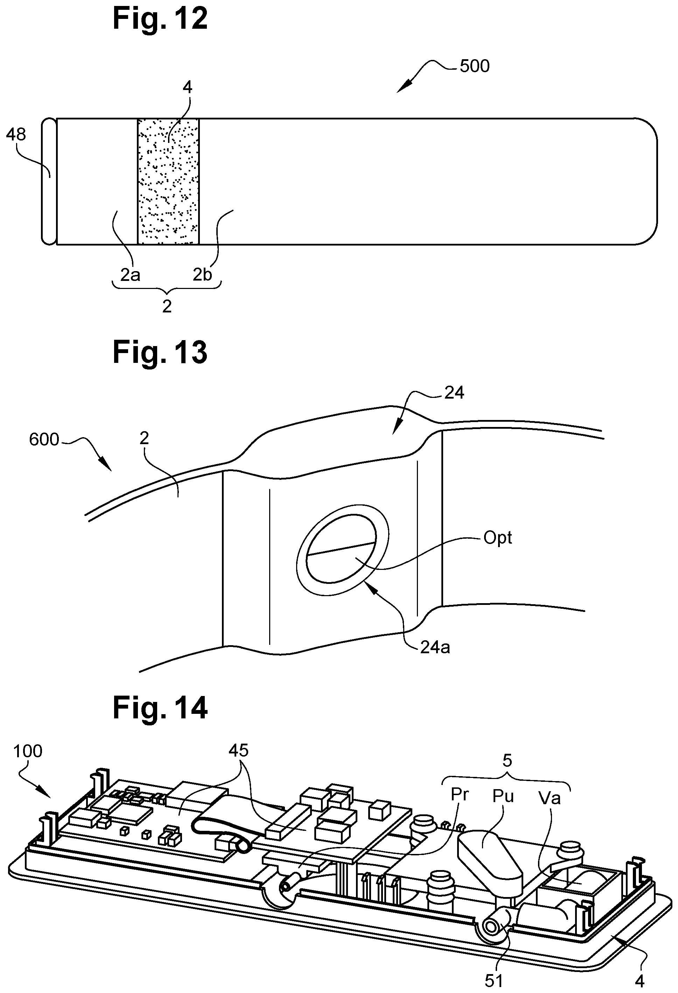

Other configurations of the scratch band or alternative attachments may be used. In particular, a buckle may be present at the first extremity 21a of the cuff 2 (such type of attachment is described in greater details with the embodiments of Figures 11 and 12).

When the device 100 is assembled, the cuff 2 preferably defines an inner volume isolated from the outside in a water leak tight manner. This is particularly advantageous when using the device in an environment where the device may be in contact with liquids or contaminants, such as a hospital.

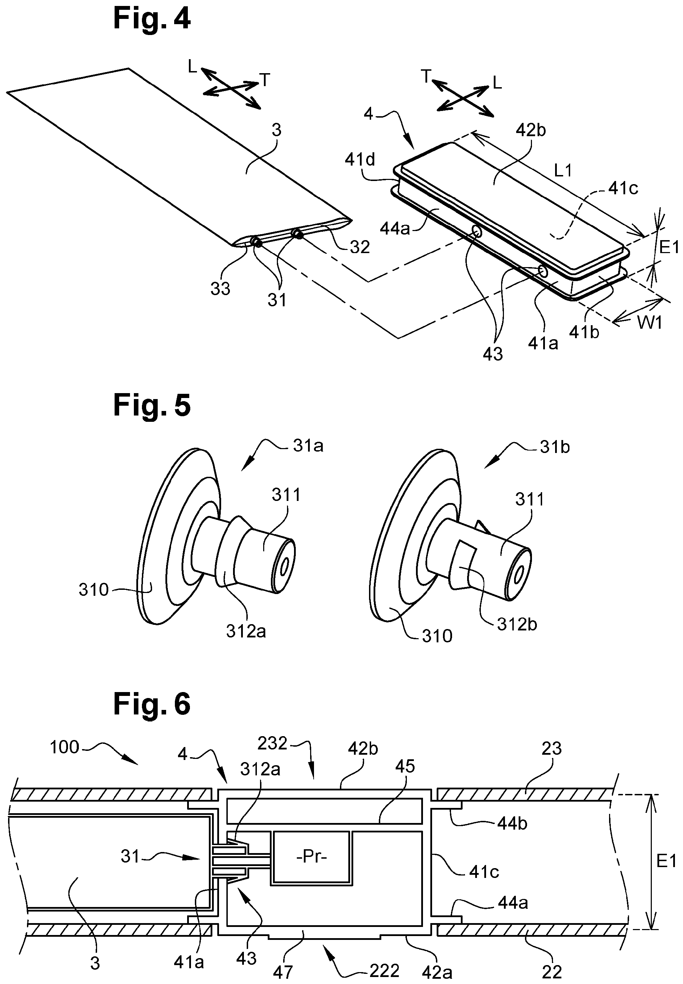

**Figure 4** shows an example of casing 4 and a bladder 3, and the pneumatic connections therebetween. The bladder 3 is configured to be inflated or deflated. In this example, the bladder 3 comprises two pneumatic connectors 31 extending from a wall 32 of the bladder, said wall 32 being transversal to the longitudinal direction L in the assembled device 100. It is understood that the wall 32 may have a curved shape and constitute a portion of the bladder 3 whose surface is transverse to the longitudinal direction L at least locally along a line extending in the transverse direction T.

The bladder 3 may be made from at least one sheet of material, for example the material may comprise a thermoplastic polyurethane (TPU) or an equivalent material. The sheet may then be folded back over itself and soldered at its edges, for example by ultrasound soldering. In the example shown, the pneumatic connectors 32 may be disposed along the folding line 33 to improve strength of the bladder and pneumatic connection. In a variant, two sheets of material may be used. In an embodiment, the bladder may be preformed to a curved shape to put it easily on the arm of the user. In an embodiment, the bladder 3 may also comprise an external layer of textile to improve its robustness.

The casing may comprise any kind of solid or flexible material, for example: polypropylene, ABS, PVC, Polyamide, rubber, resin, silicone, urethane, nylon, glass (e.g Gorilla^{®}), titanium, etc.

In this example, the casing 4 is closed, and of parallelepipedal shape (other shapes may be used with different edges or corners shapes). The casing 4 thus comprises four lateral walls 41a, 64b, 41c, 41d, a bottom wall 42a and an upper wall 42b. First and second lateral walls 41a, 41c of the casing 4 are transversal to the longitudinal direction L in the assembled device 100. The first 41a and second 42c lateral walls face each other and are connected to each other by the bottom wall 42a. The first lateral wall 41a comprises two holes 43 for the pneumatic connectors 31 of the bladder 3 to go through the casing 4. In other words, the holes 43 are configured to receive the pneumatic connectors 31. When assembled, the wall 32 of the bladder 3 bears against the lateral wall 41a of the casing 4. The use of two connectors spaced apart allows a better stability of the bladder 3 in the cuff 4 and reduces unwanted movements or rotations of the bladder 3 in the cuff. In an alternate embodiment, the bladder 3 only comprises one single pneumatic connector and the wall 41a comprises one single corresponding hole.

The cuff extends in the longitudinal direction L from an attachment edge connected to the first lateral wall 41a of the casing 4, the second lateral wall of the casing being configured to secure a part of the cuff 2, for example an opposite attachment edge, distant from the attachment edge in the longitudinal direction. In this example, the casing 4 comprises two peripheral flanges: a bottom peripheral flange 44a around the bottom wall 42a and an upper peripheral flange 44b around the upper wall 42b. Depending on the embodiment, the peripheral flanges 44a and 44b may be present only around a portion of the bottom wall 42a or the upper wall 42b, so as to connect the casing to the cuff (which could be the case in the embodiment of Figures 11 and 12).

In a variant, the casing 4 may only comprise the lateral walls 41a to 41d, i.e. the casing 4 is open at the top and the bottom and the inside of the casing is covered by the cuff 4. In a variant, the casing 4 may comprise the lateral walls 41a to 41d and only one of the bottom wall 42a or the upper wall 42b, i.e. the casing 4 is semi-open and is open at the top or the bottom, the open side of the casing 4 being covered by the cuff 4.

The casing 4 may present a length L1 ranging from 100 mm (millimeters) to 130 mm (e.g. around 120mm), a width W1 ranging from 40 mm to 50 mm (e.g. around 45 mm), and a thickness ranging from 15 mm to 30 mm (e.g. around 20 mm). The casing walls may have a thickness ranging from 1 mm to 2 mm, e.g. around 1,5 mm. The bladder 3 may have a thickness of 1 mm or less when completely deflated.

Two examples of pneumatic connectors 31a and 31b are show in **Figure 5****.** Each pneumatic connector 31a or 31b includes a circular flange 310 for positioning inside the bladder 3, a connecting pin 311 of cylindrical shape, and a retaining member 312a or 312b.

The flange 310 is configured to be positioned inside the bladder 3 and the connecting pin 311 is configured to be positioned outside the bladder 3 in order to connect the bladder 3 to a pressure management unit located inside the casing 4. The flange 310 may be tightly attached to the bladder 3, e.g., by soldering or gluing. The connecting pin 311 and the flange 310 are tightly assembled (or may form a single piece) and pierced in order to let air circulate from an opening 311a of the pin 311 to the flange 310, that is between the outside and the inside of the bladder 3.

In the first example of pneumatic connector 31a, the pin 311 comprises a retaining member or retainer 312a in the form of a ring. In the second example of pneumatic connector 31b, the pin 311 comprises a retainer 312b in the form of two radial wings. Said retainers 312a and 312b permits to secure the bladder 3 to the casing 4 as will be better described later with respect to Figure 6. Other configurations of retainers may be used to secure the bladder 3 to the casing 4.

**Figure 6** shows a cross-section view along plane VI-VI of Figure 2.

As can be seen, in this example, the lateral walls 41a to 41d of the casing 4 are located inside the cuff 2.

The casing 4 encloses a pressure management unit 5 (Figure 14), and in particular a pressure sensor Pr. The pressure sensor Pr is connected to one of the pneumatic connectors 31 of the bladder 3. The pressure sensor Pr, as other sensors or electronic components may be present on one or more Printed Circuit Board 45 (PCB).

The peripheral flanges 44a and 44b of the casing 4 are respectively bonded to the bottom layer 22 and to the upper layer 23 of the cuff 2. They ensure a smooth transition between the upper and bottom walls of the casing and the cuff 2 for better comfort of use and reduce the risk of damaging the device 100.

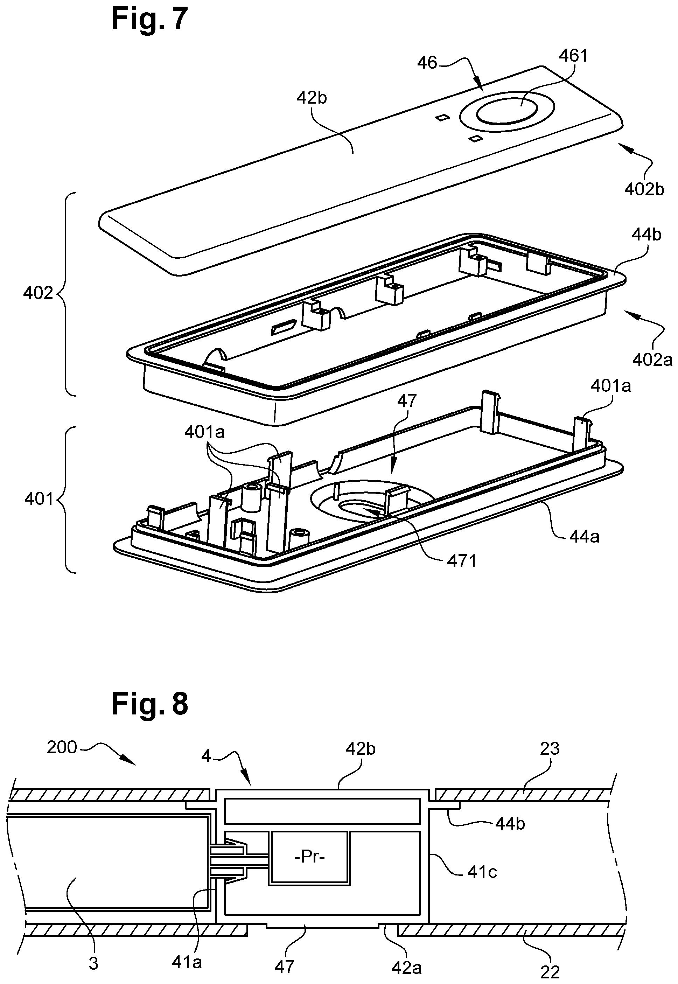

**Figure 7** shows details about the casing 4. In this example, the casing 4 comprises a bottom part 401 and an upper part 402. The upper part 402 may be also in two parts that are bonded together: a first part 402a forming the lateral walls 41a to 41d and a second part 402b forming the upper wall 42b. The bottom part 401 and the upper part 402 are assembled together using snap-fit connections 401a located inside the casing. Alternatively, other means for attaching the bottom part 401 and the upper part 402 may be used, for example using conventional screws as in the embodiment of Figures 9A and 9B.

In this example, the upper wall 42b of the casing 4 comprises an aperture 46 and a porous waterproof membrane 461 closing the aperture in order to permit gas (e.g. air) to enter or exit the casing. In other embodiments, the casing 4 may comprise one or more simple holes to allow air to enter or exit the casing.

The bottom wall 42a of the casing 4 advantageously comprises a protrusion 47 for housing an optical sensor Opt (Figure 10) and an aperture 471 in the protrusion 47 in which the optical sensor Opt can pass.

**Figure 8** shows a device 200 according to another embodiment of the invention. Compared with the embodiment of figures 1 to 6, the casing 4 of this device 200 does not comprise a bottom peripheral flange and the cuff 4 is bonded directly to the bottom wall 42a of the casing 4.

**Figure 9** and **Figure 10** show a device 300 according to another embodiment of the invention. Compared with the embodiment of figures 1 to 7, a gasket 403 is present between the bottom part 401 and the upper part 402 of the casing. In this embodiment, the upper layer 23 of the cuff 2 is bonded directly to the gasket 403. The gasket 403 may be flexible, for example made of a flexible material like rubber. Such gasket provides a better waterproofing of the cuff 2 and casing 4, and a better bond with the cuff 2 to accommodate deformations of the cuff 2 when in use.

**Figure 11** shows a device 400 according to another embodiment of the invention. In this example, the cuff 2 extends only from and is connected to one side of the casing 4. Preferably, the cuff 2 is connected to the casing 4 near the lateral wall 41a of the casing 4 where the bladder 3 is attached thereof. In this example, the casing 4 comprises a buckle 48 at the opposite side of the cuff. The buckle 48 may be integrated in the casing 4 (i.e. the casing 4 forms the buckle 48), or be a separate element from the casing 4 attached to the casing 4. The device 400 can then be attached to a limb of a user by passing the cuff through the buckle 48 and attaching the cuff 2 to itself, for example using scratch bands (not represented).

**Figure 12** shows a device 500 according to another embodiment of the invention. In this example, the cuff 2 has two distinct parts: a first part 2a extending from a first side of the casing comprising a buckle 48 at its free end, and a second part 2b extending from a second opposite side of the casing (preferably the side of the lateral wall 41a of the casing 4 where the bladder is attached thereof) enclosing the bladder 3. The buckle 48 may be integrated in the cuff 2 (i.e. be in the form of a reinforced aperture inside the first part 2b of the cuff 2), or be a separate element from the cuff. In the same manner as with device 400, the device 500 can be attached to a limb of a user by passing the second part 2b of the cuff through the buckle and attaching the second part 2b to itself, for example using scratch bands (not represented).

The devices 400 and 500 described previously each allow to attach the device to the limb of the user such that the upper wall of the casing is not covered by the cuff. This is advantageous when a display and/or a button is present on the upper wall of the casing and need to be seen or used by a user.

**Figure 13** shows a device 600 according to another embodiment of the invention. In this example, the cuff 2 presents a pocket 24 in which the casing 4 is housed (the pocket 48 can be open to insert the casing along the transversal direction, then closed). The casing 4 is thus positioned completely inside the cuff 2 or, in other words, encapsulated inside the cuff 2. The cuff 2 here comprises an aperture 24a in the pocket to let appear the optical sensor Opt of the device 600.

**Figure 14** shows several elements present inside the casing 4. In this example, the device 100 comprises a pressure management unit 5 comprising a pressure sensor Pr, a pump Pu and a valve Va. Here, the pressure sensor is connected through a first dedicated pneumatic connector to the bladder 3; and the pump Pu and the valve Va are connected together (via a Y connector 51) to a second dedicated pneumatic connector to the bladder 3. The valve Va is used to deflate the bladder. In another embodiment, the pump has an integrated valve and no other valve Va is needed.

The casing 4 comprises one or several PCB 45 to connect to different electronic elements of the device 100. One of the PCBs 45 may include a control unit for controlling the pressure management unit components and receiving signals from the sensor(s) therein, and a processing unit to perform calculations based on the measured signals. The processing unit and the control unit functions may be performed by the same electronic component or by several different electronic components.

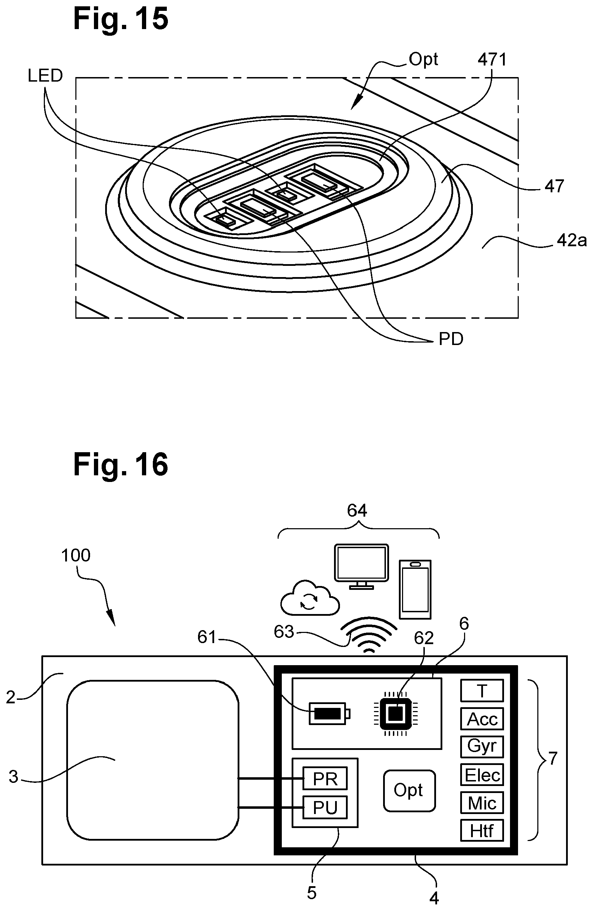

**Figure 15** shows a detailed view of the protrusion 47 of the casing 4. The aperture 471 of the protrusion 47 let appear an optical sensor Opt. The protrusion allows better contact between the skin of the user and the optical sensor Opt, thus improving the measurements. The optical sensor Opt may be a photoplethysmography sensor (PPG sensor). In this example, the optical sensor comprises two LED or LED groups (a green LED and IR+Red LEDs) and two photodetectors PD to measure the skin reflected light from the LEDs. Such an optical sensor may be used to record blood pulse features of the user, to obtain, after proper processing, biosignals like heart rate, blood pressure, pulse wave velocity, respiration rate, oxygen saturation (SpO2, SaO2), etc.

**Figure 16** is a schematic diagram of an exemplary device 100 according to the invention.

The device 100 (or any of its variants 200 to 600) comprises: a cuff, a bladder 3, and a casing 4. The casing 4 encloses a pressure management unit 5 comprising for example at least a pump Pu and a pressure sensor Pr (an optional valve Va can be used), a control and/or processing unit 6, and several other sensors 7 (e.g. an optical sensor Opt, a temperature sensor T, an accelerometer Acc, a gyroscope Gyr, an electrode Elec for ECG or EDM measurement, a microphone Mic, a heat flux sensor Htf, etc.).

For example, said pump Pu may be a rolling air pump, a piezo electric pump, a resonant gas pump, a diaphragm/membrane gas pump, and the like.

By valve it is meant any component configured to control the passage of fluid, for instance by opening, closing, or partially obstructing a pipe or an exit.

The valve Va (or a valve assembly) may be physically separated from the pump Pu. Alternatively, a valve may be integrated in the pump. For instance, the pump may comprise one or more blowers, which allows to discharge air, thereby providing a passive type valve function.

The valve Va may be a normally-open or normally-closed valve. When a valve Va is used, it is preferably a normally-closed valve. This type of valve allows to provide a constant pressure inside the inflatable compartment while reducing energy consumption.

Moreover, the pump may comprise two distinct components, one of which is configured to increase the pressure in the bladder, and the other one configured to release pressure. Alternatively, the pressurization and discharge components can be combined in a single pump.

A battery 61 may be used to supply energy to any electronic part of the device. The battery 61 is preferably a wirelessly rechargeable battery (e.g. by induction charging). A memory 62 may be used to store the acquired signals and/or results of calculations of biosignals before their transmission.

The device 100 may further have wireless connectivity capabilities (e.g. Bluetooth^{®}, BLE, 3G, 4G-LTE, LTE-M, WIFI, NFC, etc.) to send and receive acquired signals or information to and from remote devices (mobile phone, tablet, computer, cloud server, etc.). NFC technology, BLE or equivalent may be use to start a measurement, retrieve patient information and/or transmit the results to a nearby device wirelessly.

The device 100 is able to perform a blood pressure measurement directly at the limb of the subject by any method known based on pressure regulation, for example using the oscillometric method during inflation or deflation of the bladder 3.

In the examples shown, the device does not comprise any display such that the device is very discrete.

In an embodiment not illustrated, the upper wall 42b of the casing may comprise a display (e.g. LED matrix, LCD panel, LED segments, etc.) and/or one or several buttons for the user to interact with the device. When a display is present, the display may have touch sensitive functions to interact with the user. In an embodiment not illustrated, the device may comprise a speaker (e.g. for alert or alarm functions). In an embodiment not illustrated, the device may comprise a microphone (e.g. for voice control).

Such a device 100 can be useful to perform continuous monitoring of a patient, in particular its blood pressure. Indeed, the device can measure absolute blood pressure from the pressure management unit and also, when an optical sensor is included, a relative blood pressure value which can be regularly calibrated from the absolute blood pressure value. Thus, the device can perform continuous and very accurate monitoring of the blood pressure without the need to perform an absolute measure very often.

A device according to the invention is thus very convenient to use, compact and versatile. It can integrate other sensors at the limb of the subject that may contact the skin of the subject without needing too much space or thickness.

One person skilled in the art can easily understand that various features of the described embodiments can be used in combination, unless otherwise specified.

## Claims

1. A wearable blood pressure monitoring device (100; 200; 300; 400; 500; 600) comprising:
- a cuff (2) configured to be worn around a limb of a user, said cuff extending along a longitudinal direction (L);
- an inflatable compartment (3) located in the cuff and configured to be inflated or deflated; and
- a casing (4) connected to the cuff and comprising a pressure management unit (5) configured to inflate or deflate the inflatable compartment and measure a pressure therein,
**characterized in that** the inflatable compartment has a wall (32) transversal to the longitudinal direction (L) and the casing has a lateral wall (41a) transversal to the longitudinal direction (L) comprising at least one hole (43), the wearable blood pressure monitoring device (100; 200; 300; 400; 500; 600) further comprising at least one pneumatic connector (31) extending parallel to the longitudinal direction (L) between the wall (32) of the inflatable compartment and the hole (43) of the lateral wall (41a) of the casing (4) to connect the inflatable compartment to the pressure management unit (5).

2. The wearable blood pressure monitoring device according to claim 1, wherein the casing (4) houses a processing unit and at least one sensor configured to measure a physiological parameter of the user, and wherein the cuff (2) comprises a bottom side (22a) configured to face the limb of the user and the casing (4) has a bottom wall (42a) extending transversally to the lateral wall (41a), the casing (4) being attached to the cuff (2) such that at least a part of the bottom wall (42) of the casing (4) is flush with the bottom side (22a) of the cuff (2) so as to be in contact with the limb of the user when the wearable blood pressure monitoring device is worn.

3. The wearable blood pressure monitoring device according to claim 2, wherein casing (4) comprises first (41a) and second (41c) lateral walls facing each other and connected to each other by the bottom wall (42a), and the cuff extends in the longitudinal direction (L) from an attachment edge connected to the first lateral wall (41a) of the casing (4), the second lateral wall of the casing being configured to secure a part of the cuff (2) distant from the attachment edge in the longitudinal direction (L).

4. The wearable blood pressure monitoring device according to any of claims 2 and 3, wherein the bottom wall has an aperture (471) and said at least one sensor comprises an optical sensor (Opt) arranged in the casing (4) in correspondence with the aperture (471) so as to acquire a signal representative of blood pulse features at the limb of the user.

5. The wearable blood pressure monitoring device according to claim 4, wherein the bottom wall comprises a protrusion configured to house the optical sensor, the aperture being arranged in the protrusion.

6. The wearable blood pressure monitoring device according to any of claims 2 to 5, wherein the processing unit is configured to calculate a blood pressure of the user.

7. The wearable blood pressure monitoring device according to any of claims 2 to 6, wherein said at least one sensor comprises at least one of: an accelerometer (Acc), a gyroscope (Gyr), at least one electrode (Elec), an ultrasound sensor, a tomographic sensor, an acoustic sensor (Mic), a magnetometer, a humidity sensor, a temperature sensor (T), a heat-flux sensor (Htf).

8. The wearable blood pressure monitoring device according to any of claims 1 to 7, wherein the pneumatic connector (31) has a retainer (312a; 312b) cooperating with the hole (43) so as to secure the inflatable compartment (3) to the casing (4).

9. The wearable blood pressure monitoring device according to any of claims 1 to 8, wherein the wall (32) of the inflatable compartment (3) faces the lateral wall (41a) of the casing.

10. The wearable blood pressure monitoring device according to any of claims 1 to 9, wherein the inflatable compartment (3) comprises two pneumatic connectors (31) spaced apart in a transversal direction (T) and the casing (4) comprises two corresponding holes (43).

11. The wearable blood pressure monitoring device according to claim 10, wherein the pressure management unit (5) comprises a pressure sensor (Pr) connected to one of the two pneumatic connectors (32), a pump (Pu) connected to the other of the two pneumatic connectors (32), and a control unit (6) for acquiring signals from the pressure sensor and for controlling the pump.

12. The wearable blood pressure monitoring device according to any of claims 1 to 11, wherein the inflatable compartment (3) is made from a single sheet of material bent back on itself and bonded along its edges, the at least one pneumatic connector (32) being located near or on a folding line (33) of said sheet.

13. The wearable blood pressure monitoring device according to any of claims 1 to 12, wherein the cuff (2) comprises two layers (22, 23) of material bonded together along their edges so as to form an internal volume housing the inflatable compartment (3) and the casing (4).

14. A method of monitoring blood pressure of a user comprising:
- providing a wearable blood pressure monitoring device (100; 200; 300; 400; 500; 600) according to any of claims 1 to 13,
- attaching said device to a limb of the user, and
- obtaining a blood pressure of the user using said device.

## Patentansprüche

1. Tragbare Blutdrucküberwachungsvorrichtung (100; 200; 300; 400; 500; 600), umfassend:
- eine Manschette (2), die dazu konfiguriert ist, um eine Gliedmaße eines Benutzers getragen zu werden, wobei sich die Manschette entlang einer Längsrichtung (L) erstreckt;
- ein aufblasbares Fach (3), das sich in der Manschette befindet und so konfiguriert ist, dass es aufgeblasen oder entleert werden kann; und
- ein mit der Manschette verbundenes Gehäuse (4), das eine Druckmanagementeinheit (5) umfasst, die dazu konfiguriert ist, das aufblasbare Fach aufzublasen oder zu entleeren und einen Druck darin zu messen,
**dadurch gekennzeichnet, dass** das aufblasbare Fach eine Wand (32) quer zu der Längsrichtung (L) aufweist und das Gehäuse eine Seitenwand (41a) quer zu der Längsrichtung (L) aufweist, die mindestens ein Loch (43) umfasst, wobei die tragbare Blutdrucküberwachungsvorrichtung (100; 200; 300; 400; 500; 600) weiter mindestens einen pneumatischen Anschluss (31) umfasst, der sich parallel zu der Längsrichtung (L) zwischen der Wand (32) des aufblasbaren Fachs und dem Loch (43) der Seitenwand (41a) des Gehäuses (4) erstreckt, um das aufblasbare Fach mit der Druckmanagementeinheit (5) zu verbinden.

2. Tragbare Blutdrucküberwachungsvorrichtung nach Anspruch 1, wobei das Gehäuse (4) eine Verarbeitungseinheit und mindestens einen Sensor aufnimmt, der zum Messen eines physiologischen Parameters des Benutzers konfiguriert ist, und wobei die Manschette (2) eine Unterseite (22a) umfasst, die so konfiguriert ist, dass sie der Gliedmaße des Benutzers zugewandt ist, und das Gehäuse (4) eine untere Wand (42a) aufweist, die sich quer zu der Seitenwand (41a) erstreckt, wobei das Gehäuse (4) an der Manschette (2) befestigt ist, sodass mindestens ein Teil der unteren Wand (42) des Gehäuses (4) bündig mit der Unterseite (22a) der Manschette (2) abschließt, sodass sie in Kontakt mit der Gliedmaße des Benutzers steht, wenn die tragbare Blutdrucküberwachungsvorrichtung getragen wird.

3. Tragbare Blutdrucküberwachungsvorrichtung nach Anspruch 2, wobei das Gehäuse (4) erste (41a) und zweite (41c) Seitenwände umfasst, die einander gegenüberliegen und durch die untere Wand (42a) miteinander verbunden sind, und die Manschette sich in der Längsrichtung (L) von einer mit der ersten Seitenwand (41a) des Gehäuses (4) verbundenen Befestigungskante erstreckt, wobei die zweite Seitenwand des Gehäuses dazu konfiguriert ist, einen Teil der Manschette (2) zu sichern, der in der Längsrichtung (L) von der Befestigungskante entfernt ist.

4. Tragbare Blutdrucküberwachungsvorrichtung nach einem der Ansprüche 2 und 3, wobei die untere Wand eine Öffnung (471) aufweist und der mindestens eine Sensor einen optischen Sensor (Opt) umfasst, der in Übereinstimmung mit der Öffnung (471) in dem Gehäuse (4) angeordnet ist, um ein Signal zu erfassen, das für Blutpulsmerkmale an der Gliedmaße des Benutzers repräsentativ ist.

5. Tragbare Blutdrucküberwachungsvorrichtung nach Anspruch 4, wobei die untere Wand einen Vorsprung umfasst, der dazu konfiguriert ist, den optischen Sensor aufzunehmen, wobei die Öffnung in dem Vorsprung angeordnet ist.

6. Tragbare Blutdrucküberwachungsvorrichtung nach einem der Ansprüche 2 bis 5, wobei die Verarbeitungseinheit dazu konfiguriert ist, einen Blutdruck des Benutzers zu berechnen.

7. Tragbare Blutdrucküberwachungsvorrichtung nach einem der Ansprüche 2 bis 6, wobei der mindestens eine Sensor mindestens eines von einem Beschleunigungsmesser (Acc), einem Gyroskop (Gyr), mindestens einer Elektrode (Elec), einem Ultraschallsensor, einem tomografischen Sensor, einem akustischen Sensor (Mikrofon), einem Magnetometer, einem Feuchtigkeitssensor, einem Temperatursensor (T), einem Wärmestromsensor (Htf) umfasst.

8. Tragbare Blutdrucküberwachungsvorrichtung nach einem der Ansprüche 1 bis 7, wobei der pneumatische Anschluss (31) eine Halterung (312a; 312b) aufweist, die mit dem Loch (43) zusammenwirkt, um das aufblasbare Fach (3) an dem Gehäuse (4) zu sichern.

9. Tragbare Blutdrucküberwachungsvorrichtung nach einem der Ansprüche 1 bis 8, wobei die Wand (32) des aufblasbaren Fachs (3) der Seitenwand (41a) des Gehäuses zugewandt ist.

10. Tragbare Blutdrucküberwachungsvorrichtung nach einem der Ansprüche 1 bis 9, wobei das aufblasbare Fach (3) zwei in Querrichtung (T) voneinander beabstandete pneumatische Anschlüsse (31) umfasst und das Gehäuse (4) zwei entsprechende Löcher (43) umfasst.

11. Tragbare Blutdrucküberwachungsvorrichtung nach Anspruch 10, wobei die Druckmanagementeinheit (5) einen Drucksensor (Pr), der mit einem der beiden pneumatischen Anschlüsse (32) verbunden ist, eine Pumpe (Pu, die mit dem anderen der beiden pneumatischen Anschlüsse (32) verbunden ist, und eine Steuereinheit (6) zum Erfassen von Signalen von dem Drucksensor und zum Steuern der Pumpe umfasst.

12. Tragbare Blutdrucküberwachungsvorrichtung nach einem der Ansprüche 1 bis 11, wobei das aufblasbare Fach (3) aus einem einzigen Blech aus Material besteht, das auf sich selbst zurückgebogen und entlang seiner Ränder verklebt ist, wobei sich der mindestens eine pneumatische Anschluss (32) in der Nähe oder auf einer Faltlinie (33) des Blechs befindet.

13. Tragbare Blutdrucküberwachungsvorrichtung nach einem der Ansprüche 1 bis 12, wobei die Manschette (2) zwei Schichten (22, 23) von Material umfasst, die entlang ihrer Ränder miteinander verbunden sind, um ein Innenvolumen zu bilden, das das aufblasbare Fach (3) und das Gehäuse (4) aufnimmt.

14. Verfahren zum Überwachen des Blutdrucks eines Benutzers, umfassend:
- Bereitstellen einer tragbaren Blutdrucküberwachungsvorrichtung (100; 200; 300; 400; 500; 600) nach einem der Ansprüche 1 bis 13,
- Befestigen der Vorrichtung an einer Gliedmaße des Benutzers, und
- Ermitteln eines Blutdrucks des Benutzers mithilfe der Vorrichtung.

## Revendications

1. Un dispositif portable de surveillance de la pression artérielle (100 ; 200 ; 300 ; 400 ; 500 ; 600) comprenant :
• un brassard (2) configuré pour être porté autour d'un membre d'un utilisateur, ledit brassard s'étendant selon une direction longitudinale (L) ;
• un compartiment gonflable (3) situé dans le brassard et configuré pour être gonflé ou dégonflé ; et
• un boîtier (4) connecté au brassard et comprenant une unité de gestion de la pression (5) configurée pour gonfler ou dégonfler le compartiment gonflable et mesurer une pression à l'intérieur de celui-ci,
**caractérisé en ce que** le compartiment gonflable présente une paroi (32) transversale à la direction longitudinale (L) et le boîtier comporte une paroi latérale (41a) transversale à la direction longitudinale (L) incluant au moins un trou (43), le dispositif portable de surveillance de la pression artérielle (100 ; 200 ; 300 ; 400 ; 500 ; 600) comprenant en outre au moins un connecteur pneumatique (31) s'étendant parallèlement à la direction longitudinale (L) entre la paroi (32) du compartiment gonflable et le trou (43) de la paroi latérale (41a) du boîtier (4) pour connecter le compartiment gonflable à l'unité de gestion de la pression (5).

2. Le dispositif portable de surveillance de la pression artérielle selon la revendication 1, dans lequel le boîtier (4) loge une unité de traitement et au moins un capteur configuré pour mesurer un paramètre physiologique de l'utilisateur, et dans lequel le brassard (2) comprend un côté inférieur (22a) configuré pour faire face au membre de l'utilisateur et le boîtier (4) présente une paroi inférieure (42a) s'étendant transversalement par rapport à la paroi latérale (41a), le boîtier (4) étant fixé au brassard (2) de telle sorte qu'au moins une partie de la paroi inférieure (42) du boîtier (4) soit affleurante avec le côté inférieur (22a) du brassard (2) afin d'être en contact avec le membre de l'utilisateur lorsque le dispositif portable de surveillance de la pression artérielle est porté.

3. Le dispositif portable de surveillance de la pression artérielle selon la revendication 2, dans lequel le boîtier (4) comprend des premières (41a) et deuxièmes (41c) parois latérales se faisant face et reliées entre elles par la paroi inférieure (42a), et le brassard s'étend selon la direction longitudinale (L) à partir d'un bord de fixation connecté à la première paroi latérale (41a) du boîtier (4), la deuxième paroi latérale du boîtier étant configurée pour sécuriser une partie du brassard (2) distante du bord de fixation dans la direction longitudinale (L).

4. Le dispositif portable de surveillance de la pression artérielle selon l'une quelconque des revendications 2 et 3, dans lequel la paroi inférieure possède une ouverture (471) et ledit au moins un capteur comprend un capteur optique (Opt) disposé dans le boîtier (4) en correspondance avec l'ouverture (471) de manière à acquérir un signal représentatif de caractéristiques du pouls sanguin au niveau du membre de l'utilisateur.

5. Le dispositif portable de surveillance de la pression artérielle selon la revendication 4, dans lequel la paroi inférieure comprend une protubérance configurée pour loger le capteur optique, l'ouverture étant disposée dans la protubérance.

6. Le dispositif portable de surveillance de la pression artérielle selon l'une quelconque des revendications 2 à 5, dans lequel l'unité de traitement est configurée pour calculer une pression artérielle de l'utilisateur.

7. Le dispositif portable de surveillance de la pression artérielle selon l'une quelconque des revendications 2 à 6, dans lequel ledit au moins un capteur comprend l'un ou plusieurs parmi : un accéléromètre (Acc), un gyroscope (Gyr), au moins une électrode (Elec), un capteur à ultrasons, un capteur tomographique, un capteur acoustique (Mic), un magnétomètre, un capteur d'humidité, un capteur de température (T), un capteur de flux thermique (Htf).

8. Le dispositif portable de surveillance de la pression artérielle selon l'une quelconque des revendications 1 à 7, dans lequel le connecteur pneumatique (31) comporte un système de retenue (312a ; 312b) coopérant avec le trou (43) de manière à fixer le compartiment gonflable (3) au boîtier (4).

9. Le dispositif portable de surveillance de la pression artérielle selon l'une quelconque des revendications 1 à 8, dans lequel la paroi (32) du compartiment gonflable (3) fait face à la paroi latérale (41a) du boîtier.

10. Le dispositif portable de surveillance de la pression artérielle selon l'une quelconque des revendications 1 à 9, dans lequel le compartiment gonflable (3) comprend deux connecteurs pneumatiques (31) espacés dans une direction transversale (T) et le boîtier (4) comporte deux trous (43) correspondants.

11. Le dispositif portable de surveillance de la pression artérielle selon la revendication 10, dans lequel l'unité de gestion de la pression (5) comprend un capteur de pression (Pr) connecté à l'un des deux connecteurs pneumatiques (32), une pompe (Pu) connectée à l'autre des deux connecteurs pneumatiques (32), et une unité de commande (6) pour acquérir des signaux provenant du capteur de pression et pour commander la pompe.

12. Le dispositif portable de surveillance de la pression artérielle selon l'une quelconque des revendications 1 à 11, dans lequel le compartiment gonflable (3) est fabriqué à partir d'une feuille unique de matériau pliée sur elle-même et collée sur ses bords, ledit au moins un connecteur pneumatique (32) étant situé près ou sur une ligne de pliage (33) dudit feuillet.

13. Le dispositif portable de surveillance de la pression artérielle selon l'une quelconque des revendications 1 à 12, dans lequel le brassard (2) comprend deux couches (22, 23) de matériau collées ensemble le long de leurs bords de manière à former un volume interne logeant le compartiment gonflable (3) et le boîtier (4).

14. Une méthode de surveillance de la pression artérielle d'un utilisateur comprenant :
- fournir un dispositif portable de surveillance de la pression artérielle (100 ; 200 ; 300 ; 400 ; 500 ; 600) selon l'une quelconque des revendications 1 à 13,
- fixer ledit dispositif à un membre de l'utilisateur, et
- obtenir une pression artérielle de l'utilisateur en utilisant ledit dispositif
